(19) Europäisches Patentamt / European Patent Office / Office européen des brevets

(11) **EP 3 695 207 B1**

(12) **EUROPEAN PATENT SPECIFICATION**

(45) Date of publication and mention
of the grant of the patent:
**30.07.2025   Bulletin 2025/31**

(21) Application number: **18779061.3**

(22) Date of filing: **22.08.2018**

(51) International Patent Classification (IPC):
**G01N 19/02** (2006.01)    **G01N 33/483** (2006.01)
**A45D 44/00** (2006.01)

(52) Cooperative Patent Classification (CPC):
**G01N 19/02; G01N 33/4833; A45D 2044/007**

(86) International application number:
**PCT/JP2018/031846**

(87) International publication number:
**WO 2019/054175 (21.03.2019 Gazette 2019/12)**

(54) **DEVICE FOR MEASURING FRICTIONAL PROPERTIES OF FIBERS**

VORRICHTUNG ZUR MESSUNG DER REIBUNGSEIGENSCHAFTEN VON FASERN

DISPOSITIF DE MESURE DES PROPRIÉTÉS DE FROTTEMENT DE FIBRES

(84) Designated Contracting States:
**AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2017   JP 2017177831**

(43) Date of publication of application:
**19.08.2020   Bulletin 2020/34**

(73) Proprietor: **L'OREAL**
**75008 Paris (FR)**
Designated Contracting States:
**AT BE BG CH CY CZ DE DK EE ES FI FR GB GR HR HU IE IS IT LI LT LU LV MC MK MT NL NO PL PT RO RS SE SI SK SM TR**

(72) Inventors:
• **ITAYA, Ayako**
**Kawasaki-shi**
**Kanagawa 213-0012 (JP)**

• **KAMIGORI, Akiko**
**Kawasaki-shi**
**Kanagawa 213-0012 (JP)**
• **NOMURA, Mariko**
**Kawasaki-shi**
**Kanagawa 213-0012 (JP)**
• **NOMURA, Shuhei**
**Tokyo 104-0032 (JP)**
• **NOMURA, Toshio**
**Tokyo 104-0032 (JP)**

(74) Representative: **Ipsilon**
**11 rue Saint-Georges**
**75009 Paris (FR)**

(56) References cited:
**JP-A- 2012 239 615     US-A1- 2003 226 397**
**US-A1- 2016 022 022**

EP 3 695 207 B1

## Description

[TECHNICAL FIELD]

The present invention relates to a device for measuring the frictional properties of natural or synthetic fibers, for example, human hair, and in particular, to a hand-portable device therefor intended to be used, for example, in a laboratory or a hair salon.

[BACKGROUND ART]

Cosmetic hair treatments, that is, bleaching, perming, straightening, etc. are known to slightly alter the hair surface properties, by modifying the orientation (lifting up) of the cuticular scales of the hair, thereby increasing the frictional force encountered by, for example, a hair professional when pinching the hair and sliding it between his/her fingers. In order to regain smoothness, the hair having an increased frictional force, needs to be appropriately treated according to its degree of smoothness, and for this purpose it is important to measure the frictional properties of the surface of the hair. Also, the measurement of the frictional properties is important in order to know to what extent the treated hair has recovered its smoothness. Furthermore, the measurement of the frictional properties is also useful in the laboratory for specifying frictional homogeneity or heterogeneity of the hair.

Usually, such frictional properties are roughly determined by the feel of the hair. However, if a higher measurement accuracy is required, a stick-like measuring device with a sensor unit mounted at the tip thereof is used. Measurement of the frictional properties of the hair, for example, hair growing on the scalp, using a conventional device is carried out by pressing the sensor unit of the device against the hair and moving the device downward from the top of the head at a constant speed. At that time, the sensor unit measures the load applied to the unit by the operator, i.e., the pressing force and the frictional force generated during this movement. The frictional properties of the hair, in particular, the frictional coefficient thereof, is calculated by dividing the frictional force by the pressing force.

A disadvantage of the conventional device is that it is difficult to constantly apply a constant pressing force to the hair, in other words, to control the pressing force during measurement. This is because the pressing force is exclusively applied to the hair depending on the force sensation of the operator. Furthermore, when measuring the frictional properties of the hair growing on the scalp, since the orientation of the device continuously changes during measurement, it is more difficult to constantly apply a constant pressing force to the measurement target. For this reason, it is not possible to realize high repeatability and reproducibility by using the conventional device.

Examples of prior art related to the present invention are disclosed in the following publications. JP2004-158526 discloses a hair damage measurement system by a frequency fluctuation analysis on a hair comb. JP2004-159830 discloses a hair damage measurement system with a microphone on a hair comb. JP2009-201812 discloses a hair characteristic evaluation system with a piezoelectric sensor (PVDF film) on a hair comb. JP2012239615 discloses a hair brush having a control unit which is controlled according to friction coefficient detected by friction sensor to change output of ventilation unit and heating unit.

[DISCLOSURE OF THE INVENTION]

In view of the above, an object of the present invention is to provide a novel device for measuring the frictional properties of natural or synthetic fibers which can overcome the disadvantage of the prior art device. In particular, an object of the present invention is to provide a novel device for measuring the frictional properties of fibers which can apply a constant load to the fibers whose frictional properties are to be measured during the measurement, thereby realizing high repeatability and reproducibility.

In order to achieve this object, the present invention provides a device according to claim 1.

The device according to the present invention configured as described above includes a nipping system that can constantly apply a constant pressing force (load) to the fibers whose frictional propertieptusions are to be measured. Therefore, the frictional forces arising when the device is being moved relative to the fibers sandwiched between the sensor unit and the nipping member in a direction crossing the longitudinal axis of the base member can be measured in a state in which a constant pressing force is applied to the fibers by this nipping system. Thus, the device according to the present invention is operator-independent. That is, the device according to the present invention can eliminate variations in the pressing force applied to the fibers, so that it becomes possible to measure the frictional properties of the fibers accurately and with high repeatability and reproducibility. Furthermore, in the device according to the present invention, unlike conventional fiber-pressing type devices, the frictional properties are measured only for fibers loaded in the nipping system. Therefore, the number of fibers to be measured can be freely controlled.

The term "fibers" as used herein is intended to encompass naturally-derived fibers such as hair, wool, silk, and cotton, or synthetic fibers such as nylon, polyester, polyamide, and carbon. In addition, the term "fibers" as used herein is intended to encompass woven or knitted fibers such as ropes, cables, cloth, and paper.

According to one preferred aspect of the present invention, the nipping member may be connected to the base member such that the one end of the nipping member is separated from the sensor unit by pressing the other end of the nipping member which is present on the side opposite to the one end of the nipping member. This is useful for enhancing the operability of the device.

According to the present invention, the nipping member comprises first and second protrusions on its face facing the sensor unit at the one end of the nipping member, and the first and second protrusions are arranged such that the sensor unit is located between the first protrusion and the second protrusion when the nipping member is closed to apply the pressing force on the fibers sandwiched between the sensor unit and the nipping member. In this aspect, the first and second protrusions serve as a guide for the fibers (in particular a bundle of the fibers), which serves to ensure that the fibers are certainly positioned within the measurement space between the sensor unit and the nipping member.

According to one preferred aspect of the present invention, at least one spring may be interposed between the base member and the nipping member, and the spring may function to apply a constant pressing force on the fibers sandwiched between the sensor unit and the nipping member. In a particularly preferred aspect, the spring may be interchangeable to adjust the pressing force applied to the fibers.

According to one preferred aspect of the present invention, a width of a region in the base member where the sensor unit is mounted may be larger than a width of the sensor unit. This reduces the bending of the fibers at the corners of the sensor unit, which is useful for further improving the measurement accuracy.

According to one preferred aspect of the present invention, the device may be configured to be capable of wired or wireless connection to an external equipment for storing and/or processing the data outputted from the sensor unit. For the wireless connection to the external equipment, for example, Wi-Fi® or Bluetooth® can be used.

According to one preferred aspect of the present invention, at least a part of the sensor unit and/or the nipping member coming into contact with the fibers may be formed of a material that is not susceptible to generation of electrostatic forces when the device is being moved relative to the fibers. This helps to further improve the measurement accuracy.

[BRIEF DESCRIPTION OF THE DRAWINGS]

Non-limiting and representative embodiments of the present invention will now be explained in detail below referring to the attached drawings.

> Fig. 1 is a schematic diagram of a measuring system including a device for measuring frictional properties of fibers according to an embodiment of the present invention.
> Fig. 2 is a side view of the device shown in Fig. 1 with the device in a closed state.
> Fig. 3 is also a side view of the device shown in Fig. 1 with the device in an open state.

Fig. 4 is a perspective view in a disassembled state showing a structure of a connecting portion of a base member and a nipping member in the device shown in Figs. 1 to 3.
Fig. 5 is a plan view of a portion of the base member of the device shown in Figs. 1 to 4 where a sensor unit is mounted.
Fig. 6 shows a situation in which the frictional properties of hair are being measured using the device shown in Figs. 1 to 5.
Fig. 7 shows the results of the measurement of frictional properties of hair, performed by different operators using the device shown in Figs. 1 to 6 (Example 1).
Fig. 8 shows the results of the measurement of frictional properties of hair, performed on different days using the device shown in Figs. 1 to 6 (Example 2).
Fig. 9 shows the results of the measurement of frictional properties of different model's hair, performed using the device shown in Figs. 1 to 6 (Example 3).
Fig. 10 shows the results of the measurement of frictional properties of hair, performed after different numbers of times of shampooing using the device shown in Figs. 1 to 6 (Example 4).
Fig. 11 shows the results of the measurement of frictional properties of three different materials, performed using the device shown in Figs. 1 to 6 (Example 5).

[DETAILED DESCRIPTION OF EMBODIMENTS]

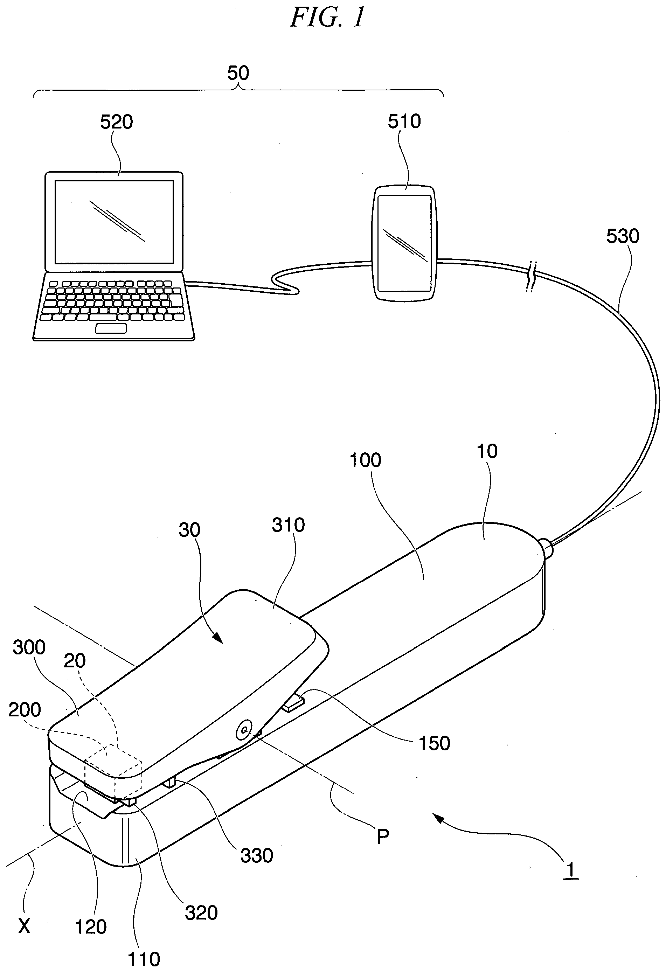

An exemplary embodiment of the present invention will now be described with reference to Figs. 1 to 6. Described below is a hand-portable device for measuring the frictional properties of human hair, more specifically for estimating the frictional coefficient thereof in a state of growing from the scalp, i.e. under "vivo" conditions such as those met in hair salons. However, the exemplary embodiment of the present invention can also be used to measure the frictional properties of hair in a cut and tensioned state, i.e. under "vitro" conditions such as those met in a laboratory. Furthermore, the exemplary embodiment of the present invention can also be used to measure the frictional properties of various natural or synthetic fibers other than hair.

Fig. 1 shows a device 1 for measuring frictional properties of fibers according to one embodiment of the present invention in a state in which the device 1 is connected to external equipment 50, that is, a data logger 510 for storing the data outputted from a sensor unit 20 (described below in more detail) of the device 1 and a personal computer 520 for processing the data. In this embodiment, the device 1 is configured to be wired to the external device 50. However, in other embodiments, the device 1 may be configured to be wirelessly connected to the external device 50 using, for example, Wi-Fi® or Bluetooth®.

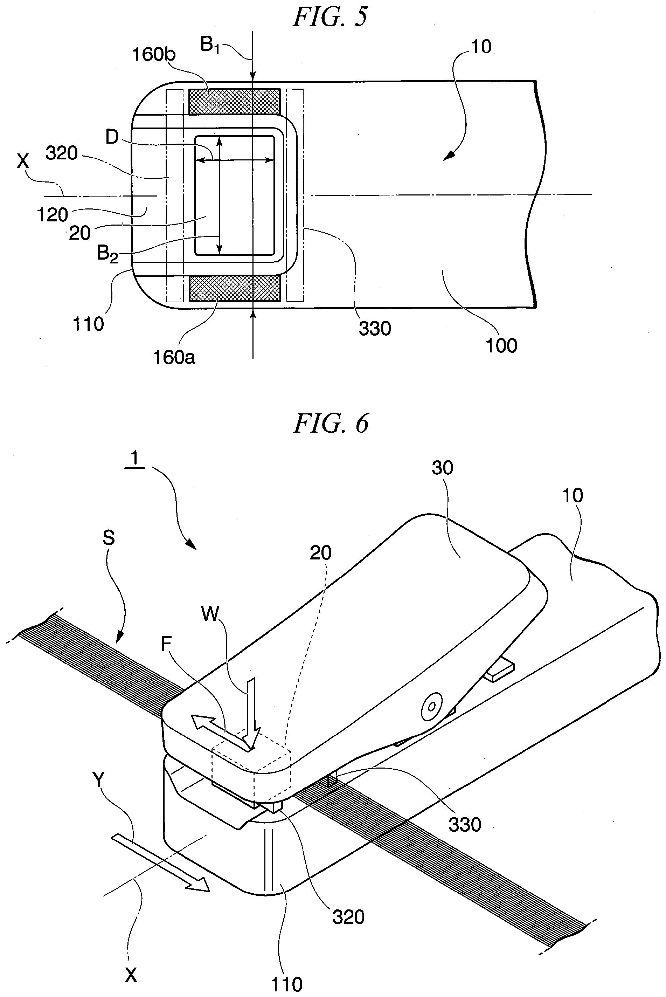

As can be seen from Fig. 1, the device 1 generally comprises: a base member 10 which functions as a handle for operation; the above mentioned sensor unit 20 mounted on a distal end (one end) 110 of the base member 10; and a nipping member 30 connected to the base member 10 so that its distal end (one end) 300 faces the sensor unit 20. In this embodiment, the distal end 300 is a solid plate shape. Further, from a proximal end of the base member 10 (not denoted by a reference numeral), a signal cable 530 connected to the external device 50 extends. Hair S (see Fig. 6), more specifically a bundle of hair whose frictional properties are to be measured, is sandwiched between the sensor unit 20 and the distal end 300 of the nipping member 30. In this device 1, the base member 10 and the nipping member 30 are formed of a neutral material (for example, a suitable plastic) which is not susceptible to generation of electrostatic forces during measurement of the frictional properties of the hair S, i.e., when the device 1 is being moved relative to the hair S. Furthermore, a part of the sensor unit 20 coming into contact with the hair S is also formed of a neutral material (for example, a suitable rubber) which is not susceptible to generation of electrostatic forces when the device 1 is being moved relative to the hair S.

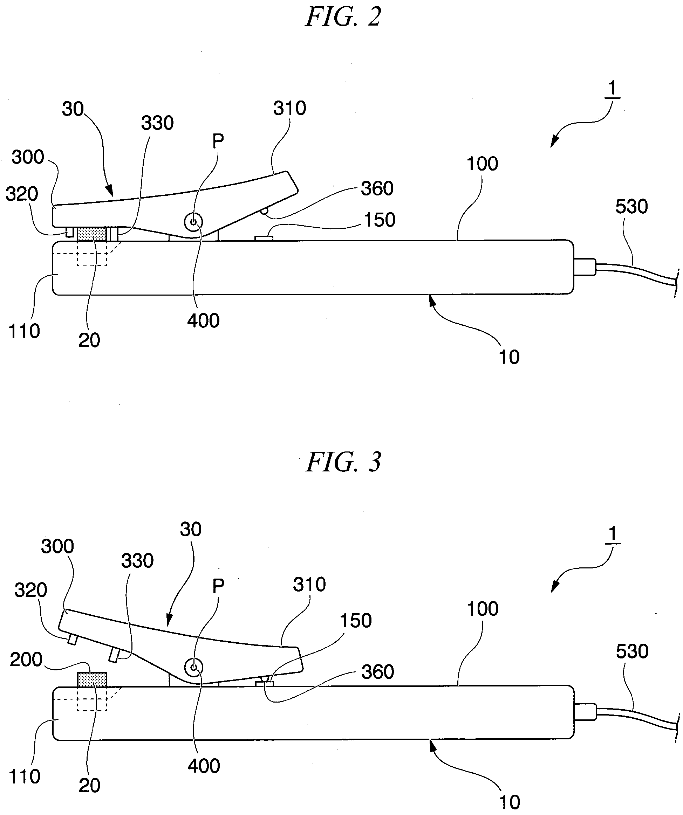

As can be seen from Fig. 1, the base member 10 has a longitudinal axis X and a top surface 100. Furthermore, as can be seen from Figs. 2 and 3 showing the device 1 viewed from the side, the sensor unit 20 is mounted on the side of the top surface 100 of the base member 10 so that a part thereof protrudes from the top surface 100. More specifically, at the distal end 110 of the base member 10, a shallow recess 120 is formed on the side of the top surface 100. The upper portion of the sensor unit 20 protrudes beyond the top surface 100 from the bottom surface of the recess 120. As the sensor unit 20, for example, a biaxial sensor unit available from Trinity-Lab. Inc. of Tokyo, Japan can be used.

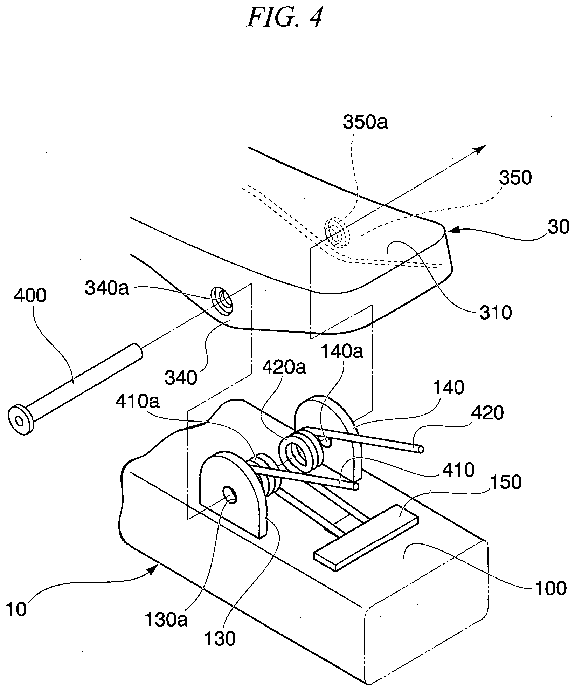

In addition to Figs. 1 to 3, as can be further seen from Fig. 4, the nipping member 30 cooperating with the sensor unit 20 is pin-connected to the base member 10 such that its distal end 300 faces an upper surface 200 of the sensor unit 20. More specifically, a pair of brackets 130, 140 are integrally formed on the top surface 100 of the base member 10, and these brackets 130, 140 have through holes 130a, 140a. On the other hand, the nipping member 30 generally has an inverted U-shaped profile in the intermediate section, and through holes 340a, 350a are formed in the opposing side walls 340, 350 constituting this inverted U-shaped profile. In particular, as can be seen from Fig. 4, a pin 400, in the assembled state of the base member 10 and the nipping member 30, extends through the holes 130a, 140a and the holes 340a, 350a and is held so as not to come off. As a result, the base member 10 and the nipping member 30 are pin-connected as described above.

Because the device 1 has such a configuration, the nipping member 30 is rotatable with respect to the base member 10 about pivot axis P above the top surface 100 of the base member 10 and perpendicular to the longitudinal axis X. In other words, the nipping member 30 is connected to the base member 10 such that the distal end 300 of the nipping member 30 is separated from the sensor unit 20 by pressing a proximal end (other end) 310 of the nipping member 30 which is present on the side opposite to the distal end 300 of the nipping member 30, that is, in a manner like a seesaw. The nipping member 30 is rotatable until a stopper 360 (see Figs. 2 and 3) projecting from the lower surface of the proximal end 310 of the nipping member 30 abuts against a stopper receiver 150 on the upper surface 100 of the base member 10.

The state where the proximal end 310 of the nipping member 30 is not pressed, that is, the closed state of the nipping member 30, is as shown in Fig. 2. On the other hand, the state where the nipping member 30 is pivoted to the final position by pressing the proximal end 310 of the nipping member 30, that is, the open state of the nipping member 30, is as shown in Fig. 3. The hair S whose frictional properties are to be measured, is loaded between the sensor unit 20 and the nipping member 30 in this open state. The sensor unit 20 measures a frictional force generated when, as shown in Fig. 6, the device 1 is being relatively moved with respect to the hair S sandwiched between the sensor unit 20 and the nipping member 30 in a direction perpendicular to the longitudinal axis X of the base member 10, i.e., in a lateral direction. The sensor unit 20 then outputs the value of the measured frictional force to the external device 50, as described above.

In the device 1 configured as described above, during the measurement of the frictional properties, the nipping member 30 applies a constant pressing force on the hair S sandwiched between the sensor unit 20 and the nipping member 30. More specifically, as can be seen from Fig. 4 showing the structure of the connecting portion of the base member 10 and the nipping member 30 in a disassembled state, in this device 1, two substantially V-shaped springs 410, 420 are interposed between the base member 10 and the nipping member 30, in particular the proximal end 310 thereof. These springs 410, 420 each include coil sections 410a, 420a at its bend and the pin 400 connecting the nipping member 30 to the base member 10 passes through these coil sections 410a, 420a. The springs 410, 420 thus arranged always apply a force pushing up the proximal end 310 of the nipping member 30 thereto. Therefore, the springs 410, 420 function to exert a constant pressing force on the hair S sandwiched between the sensor unit 20 and the nipping member 30.

In this embodiment, the spring rate of the springs 410, 420 is selected such that the nipping member 30 exerts a pressing force of about 2.5 N on the sandwiched hair S, although is not limited thereto. In addition, the springs 410, 420 can be replaced to adjust the pressing force applied to the sandwiched hair S.

The nipping member 30 comprises first and second protrusions 320, 330 on its face facing the sensor unit 20 at the distal end 300 of the nipping member 30. Both of the first and second protrusions 320, 330 have elongated flat plate shapes and are arranged parallel to each other. The first and second protrusions 320, 330 serve as a guide for the hair (especially a bundle of hair) that ensures that the hair S is located within the measurement space between the sensor unit 20 and the nipping member 30. In this embodiment, the height of the first protrusion 320 disposed on the tip side of the nipping member 30 is slightly lower than the height of the rear second protrusion 330.

As can be seen from Fig. 5 showing the portion of the base member 10 where the sensor unit 20 is provided as viewed from above, the first and second protrusions 320, 330 are arranged such that the sensor unit 20 is located between the first protrusion 320 and the second protrusion 330 when the nipping member 30 is closed to apply the pressing force on the hair S sandwiched between the sensor unit 20 and the nipping member 30. That is, the first and second protrusions 320, 330 are spaced apart by a slightly greater distance than the dimension D of the sensor unit 20 along the longitudinal axis X of the base member 10.

As can also be seen from Fig. 5, in this embodiment, a width $B_1$ of a region in the base member 10 where the sensor unit 20 is mounted is larger than a width $B_2$ of the sensor unit 20. As a result, on both sides of the sensor unit 20, support areas 160a, 160b for reducing the bending of the hair S at the corners of the sensor unit 20 is formed. More specifically, these support areas 160a, 160b are present on both sides of the shallow recess 120 of the base member 10 and are indicated by shading in Fig. 5.

Fig. 6 shows the situation in which frictional properties of the hair S are being measured using the device 1 configured as described above. Prior to the measurement, the bundle of hair S whose frictional properties are to be measured, is sandwiched between the sensor unit 20 and the distal end 300 of the nipping member 30. The amount of hair sandwiched between the sensor unit 20 and the nipping member 30 is adjustable, but in this embodiment, for example, it is about 0.1 to 2 grams, preferably 1 gram. In this nipped state, the nipping member 30 applies a constant pressing force on the hair S by the action of the spring 410, 420. Furthermore, the bundle of hair S is guided from both sides of the sensor unit 20 by the first and second protrusions 320, 330.

After the hair S is set on the device 1, it is moved by an operator, that is, manually (or automatically if necessary) in the direction of the length of the hair S, that is, in a direction Y orthogonal to the longitudinal axis X of the base member 1, at a constant speed. While the device 1 is moving in the direction Y, the sensor unit 20 measures the frictional force F generated during this movement together with a constant pressing force (vertical load) W applied to the hair S by the nipping member 30. It should be noted here that the pressing force W acts perpendicular to the upper surface 200 of the sensor unit 20, while the frictional force F occurs in a direction opposite to the direction of movement Y of the device 1 (see Fig. 6). Subsequently, the sensor unit 20 outputs the measured values of the constant pressing force W and the measured frictional force F to the external equipment 50. In this embodiment, the frictional coefficient (the dynamic frictional coefficient) $\mu$ of the hair S is calculated by the following equation in the external equipment 50:

$$\mu = F / W.$$

The value of the frictional coefficient of the hair S thus obtained is subsequently stored and/or processed by the external device 50. However, in an alternative embodiment, the frictional coefficient $\mu$ of the fiber to be measured may be directly output from the device 1 to the external device 50.

Example 1: Reproducibility of the measurement of the frictional properties (under vitro conditions)

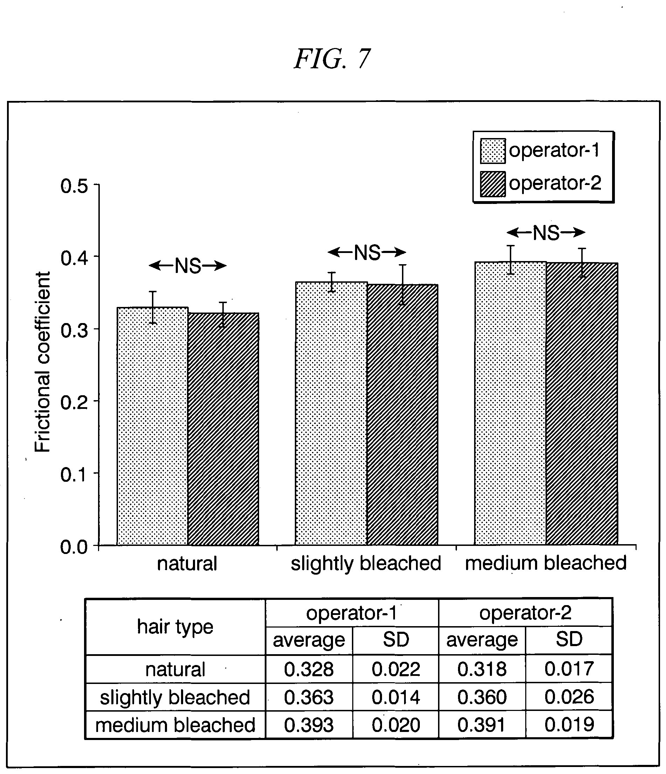

Measurement of the frictional properties of hair, more specifically, a swatch of hair, was carried out under vitro conditions by different operators, that is, operator-1 and operator-2, using the device described above. Three kinds of hair, that is, "natural", "slightly bleached", and "medium bleached" are objects to be measured. Measurement was performed a plurality of times, and an average value of the frictional coefficient and a standard deviation (SD) were calculated. The results are shown in Fig. 7. As can be seen from Fig. 7, almost irrespective of the hair quality, the standard deviation (SD) of the measured values by both operator-1 and operator-2 is very small. From this, it is understood that measurement using the device according to the embodiment of the present invention realizes high reproducibility.

Example 2: Repeatability of the measurement of the frictional properties

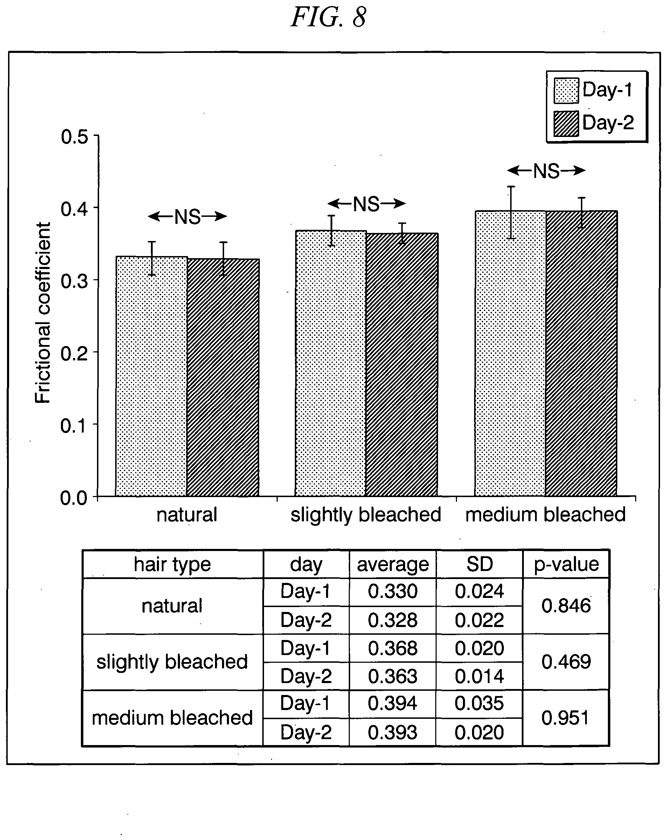

Measurement of the frictional properties of hair was carried out on successive different dates, that is, Day-1 and Day-2, using the device described above. Three kinds of hair, that is, "natural", "slightly bleached", and "medium bleached", are objects to be measured. Measurement was performed a plurality of times, and an average value of the frictional coefficient and a standard deviation (SD) were calculated. The results are shown in Fig. 8 together with p-value (Significant Probability). As can be seen from Fig. 8, almost irrespective of the hair quality, the standard deviation SD of the measured values on both Day-1 and Day-2 is very small. From this, it is understood that measurement using the device according to the embodiment of the present invention realizes high repeatability.

Example 3: Accuracy of the measurement of the frictional properties (under vivo conditions)

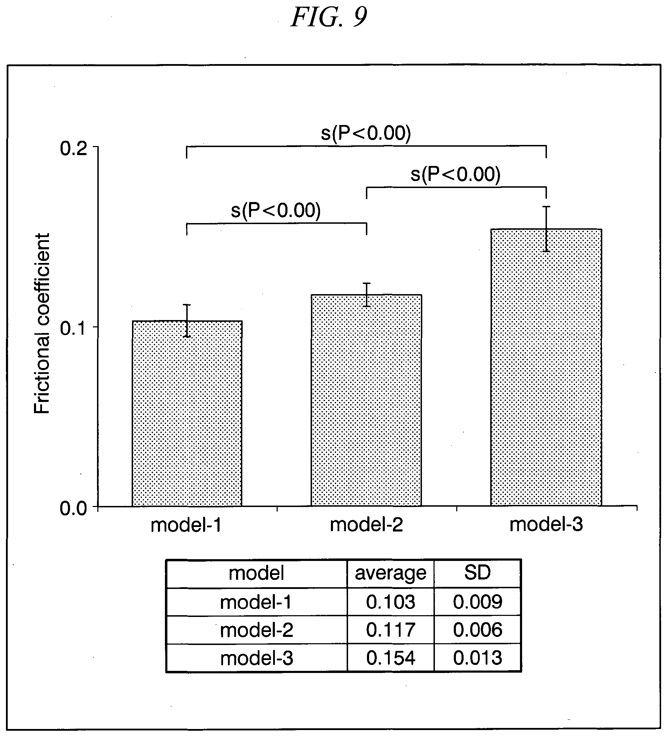

Measurement of the frictional properties of hair was carried out using the device described above. The different model's hair, that is, "model-1", "model-2", and "model-3" are objects to be measured. Measurement was performed a plurality of times, and an average value of the frictional coefficient and a standard deviation (SD) were calculated. The results are shown in Fig. 9. As can be seen from Fig. 9, almost irrespective of the hair quality, the standard deviation SD of the measured values is very small. From this, it is understood that measurement using the device according to the embodiment of the present invention realizes high accuracy under the vivo conditions.

Example 4: Accuracy of the measurement of the frictional properties (under vitro conditions)

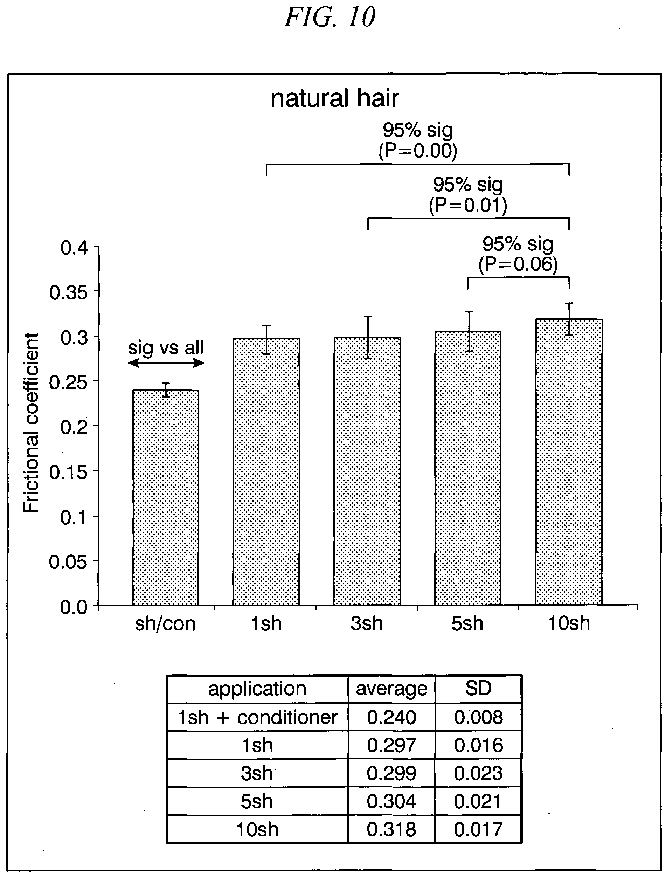

Measurement of the frictional properties of hair, more particularly a swatch of hair, was carried out under vitro conditions using the device described above. The objects to be measured are: once-washed and conditioner-applied natural hair (1sh + conditioner), once-washed natural hair (1sh), three-times-washed natural hair (3sh), five-times-washed natural hair (5sh), and 10-times-washed natural hair (10wh). Measurement was performed a plurality of times, and an average value of the frictional coefficient and a standard deviation (SD) were calculated. The results are shown in Fig. 10. As can be seen from Fig. 10, almost irrespective of the type and frequency of treatments applied to the hair, the standard deviation SD of the measured values is very small. From this, it is understood that measurement using the device according to the embodiments of the present invention also realizes high accuracy under vitro conditions.

Example 5: Accuracy of the measurement of the frictional properties (the different material fibers)

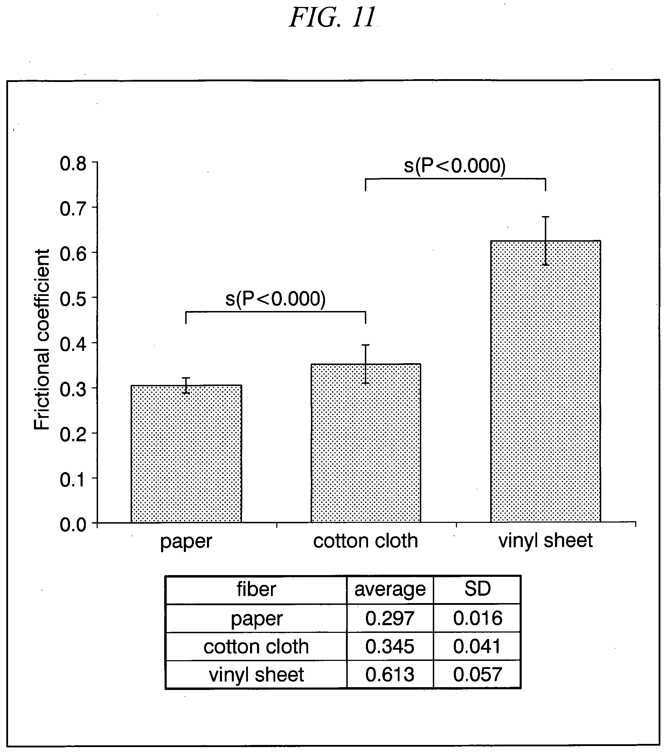

Measurement of the frictional properties of the fibers was carried out using the device described above. Three kinds of fibers, that is, "paper", "cotton cloth", and "vinyl sheet", are objects to be measured. Measurement was performed a plurality of times, and an average value of the frictional coefficient and a standard deviation (SD) were calculated. The results are shown in Fig. 11. As can be seen from Fig. 11, almost irrespective of the fiber quality, the standard deviation SD of the measured values is very small. From this, it is understood that measurement using the device according to the embodiment of the present invention realizes high accuracy on not only hair but also other fibers.

Preferred embodiments of the present invention have been explained above referring to the drawings. However, the present invention is not limited to these embodiments, and various modifications and changes may be made to the above-described embodiments without deviating from the scope of the present invention, and such modifications and changes are included in the scope of the present invention.

**Claims**

1. A device (1) for measuring frictional properties of fibers (S), the device (1) comprising:

   a base member (10) having a longitudinal axis (X) and a top surface (100);
   a sensor unit (20) mounted on a side of the top surface (100) of the base member (10) at one end (110) of the base member (10); and
   a nipping member (30) pivotably connected to the base member (10) about a pivot axis (P) above the top surface (100) of the base member (10) such that one end (300) of the nipping member (30) faces the sensor unit (20);
   wherein
   fibers (S) whose frictional properties are to be measured are sandwiched between the sensor unit (20) and the one end (300) of the nipping member (30);
   the sensor unit (20) is configured such that it measures at least a frictional force generated when the device (1) is being relatively moved with respect to the fibers (S) sandwiched between the sensor unit (20) and the nipping member (30) in a direction crossing the longitudinal axis (X) of the base member (10), and outputs the measurement data; and
   the nipping member (30) applies a constant pressing force on the fibers (S) sandwiched between the sensor unit (20) and the nipping member (30) during the measurement of the frictional force,
   the device being **characterized in that**:
   the nipping member (30) comprises first and second protrusions (320, 330) on its face facing the sensor unit (20) at the one end (300) of the nipping member (30), and wherein the first and second protrusions (320, 330) are arranged such that the sensor unit (20) is located between the first protrusion (320) and the second protrusion (330) when the nipping member (30) is closed to apply the pressing force on the fibers (S) sandwiched between the sensor unit (20) and the nipping member (30).

2. The device (1) according to claim 1, wherein the nipping member (30) is connected to the base member (10) such that the one end (300) of the nipping member (30) is separated from the sensor unit (20) by pressing the other end (310) of the nipping member (30) which is present on the side opposite to the one end (300) of the nipping member (30).

3. The device (1) according to any one of claims 1 or 2, wherein at least one spring (410, 420) is interposed between the base member (10) and the nipping member (30), and wherein the spring (410, 420) functions to apply a constant pressing force on the fibers (S) sandwiched between the sensor unit (20) and the nipping member (30).

4. The device (1) according to claim 3, wherein the spring (410, 420) is interchangeable to adjust the pressing force applied to the fibers (S).

5. The device (1) according to any one of claims 1 to 4, wherein a width ($B_1$) of a region in the base member (10) where the sensor unit (20) is mounted is larger than a width ($B_2$) of the sensor unit (20).

6. The device (1) according to any one of claims 1 to 5, wherein the device (1) is configured to be capable of wired or wireless connection to external equipment (50) for storing and/or processing the data outputted from the sensor unit (20).

7. The device (1) according to any one of claims 1 to 6, wherein at least a part of the sensor unit (20) and/or the nipping member (30) coming into contact with the fibers (S) is formed of a material that is not susceptible to generation of electrostatic forces when the device (1) is being moved relative to the fibers (S).

**Patentansprüche**

1. Vorrichtung (1) zum Messen von Reibungseigenschaften von Fasern (S), wobei die Vorrichtung (1) umfasst:

ein Basiselement (10) mit einer Längsachse (X) und einer oberen Oberfläche (100);
eine Sensoreinheit (20), die an einer Seite der oberen Oberfläche (100) des Basiselements (10) an einem Ende (110) des Basiselements (10) angebracht ist; und
ein Klemmelement (30), das mit dem Basiselement (10) um eine Schwenkachse (P) über der oberen Oberfläche (100) des Basiselements (10) schwenkbar verbunden ist, so dass ein Ende (300) des Klemmelements (30) der Sensoreinheit (20) zugewandt ist;
wobei
Fasern (S), deren Reibungseigenschaften gemessen werden sollen, zwischen der Sensoreinheit (20) und dem einen Ende (300) des Klemmelements (30) angeordnet sind;
die Sensoreinheit (20) so gestaltet ist, dass sie wenigstens eine Reibungskraft misst, die erzeugt wird, wenn die Vorrichtung (1) relativ zu den Fasern (S), die zwischen der Sensoreinheit (20) und dem Klemmelement (30) angeordnet sind, in einer Richtung bewegt wird, die die Längsachse (X) des Basiselements (10) kreuzt, und die Messdaten ausgibt; und
das Klemmelement (30) während der Messung der Reibungskraft eine konstante Presskraft auf die Fasern (S), die zwischen der Sensoreinheit (20) und dem Klemmelement (30) angeordnet sind, ausübt, wobei die Vorrichtung **dadurch gekennzeichnet ist, dass**:
das Klemmelement (30) einen ersten und einen zweiten Vorsprung (320, 330) an seiner der Sensoreinheit (20) zugewandten Seite an dem einen Ende (300) des Klemmelements (30) umfasst, und wobei der erste und der zweite Vorsprung (320, 330) so angeordnet sind, dass die Sensoreinheit (20) zwischen dem ersten Vorsprung (320) und dem zweiten Vorsprung (330) angeordnet ist, wenn das Klemmelement (30) geschlossen ist, um die Presskraft auf die Fasern (S), die zwischen der Sensoreinheit (20) und dem Klemmelement (30) angeordnet sind, auszuüben.

2. Vorrichtung (1) nach Anspruch 1, wobei das Klemmelement (30) so mit dem Basiselement (10) verbunden ist, dass das eine Ende (300) des Klemmelements (30) von der Sensoreinheit (20) durch Drücken des anderen Endes (310) des Klemmelements (30), das auf der dem einen Ende (300) des Klemmelements (30) gegenüberliegenden Seite vorhanden ist, getrennt wird.

3. Vorrichtung (1) nach einem der Ansprüche 1 oder 2, wobei wenigstens eine Feder (410, 420) zwischen dem Basiselement (10) und dem Klemmelement (30) angeordnet ist und wobei die Feder (410, 420) dazu dient, eine konstante Presskraft auf die Fasern (S), die zwischen der Sensoreinheit (20) und dem Klemmelement (30) angeordnet sind, auszuüben.

4. Vorrichtung (1) nach Anspruch 3, wobei die Feder (410, 420) ausgetauscht werden kann, um die auf die Fasern (S) ausgeübte Presskraft einzustellen.

5. Vorrichtung (1) nach einem der Ansprüche 1 bis 4, wobei eine Breite ($B_1$) eines Bereichs des Basiselements (10), in dem die Sensoreinheit (20) angebracht ist, größer als eine Breite ($B_2$) der Sensoreinheit (20) ist.

6. Vorrichtung (1) nach einem der Ansprüche 1 bis 5, wobei die Vorrichtung (1) dafür gestaltet ist, zur drahtgebundenen oder drahtlosen Verbindung mit externen Geräten (50) zum Speichern und/oder Verarbeiten der von der Sensoreinheit (20) ausgegebenen Daten fähig zu sein.

7. Vorrichtung (1) nach einem der Ansprüche 1 bis 6, wobei wenigstens ein Teil der Sensoreinheit (20) und/oder des Klemmelements (30), der mit den Fasern (S) in Kontakt kommt, aus einem Material besteht, das nicht für Erzeugung elektrostatischer Kräfte anfällig ist, wenn die Vorrichtung (1) relativ zu den Fasern (S) bewegt wird.

## Revendications

1. Dispositif (1) destiné à mesurer des propriétés de frottement de fibres (S), le dispositif (1) comprenant :

   un élément de base (10) ayant un axe longitudinal (X) et une surface supérieure (100) ;
   une unité de détection (20) montée sur un côté de la surface supérieure (100) de l'élément de base (10) à une extrémité donnée (110) de l'élément de base (10) ; et
   un élément de pincement (30) relié avec faculté de pivotement à l'élément de base (10) autour d'un axe pivot (P) au-dessus de la surface supérieure (100) de l'élément de base (10) de telle sorte qu'une extrémité donnée (300) de l'élément de pincement (30) fait face à l'unité de détection (20) ;
   dans lequel
   des fibres (S) dont les propriétés de frottement doivent être mesurées sont prises en sandwich entre l'unité de détection (20) et l'extrémité donnée (300) de l'élément de pincement (30) ;
   l'unité de détection (20) est conçue de telle sorte qu'elle mesure au moins une force de frottement générée quand le dispositif (1) est soumis à un déplacement relatif par rapport aux fibres (S) prises en sandwich entre l'unité de détection (20) et l'élément de pincement (30) dans une direction croisant l'axe longitudinal (X) de l'élément de base (10), et délivre les données de mesure ; et
   l'élément de pincement (30) applique une force de compression constante sur les fibres (S) prises en sandwich entre l'unité de détection (20) et l'élément de pincement (30) pendant la mesure de la force de frottement, le dispositif étant **caractérisé en ce que** : l'élément de pincement (30) comprend des première et deuxième saillies (320, 330) sur sa face tournée vers l'unité de détection (20) à l'extrémité donnée (300) de l'élément de pincement (30), et dans lequel les première et deuxième saillies (320, 330) sont disposées de telle sorte que l'unité de détection (20) est située entre la première saillie (320) et la deuxième saillie (330) quand l'élément de pincement (30) est fermé pour appliquer la force de compression sur les fibres (S) prises en sandwich entre l'unité de détection (20) et l'élément de pincement (30).

2. Dispositif (1) selon la revendication 1, dans lequel l'élément de pincement (30) est relié à l'élément de base (10) de telle sorte que l'extrémité donnée (300) de l'élément de pincement (30) est séparée de l'unité de détection (20) par appui sur l'autre extrémité (310) de l'élément de pincement (30) qui est présente sur le côté opposé à l'extrémité donnée (300) de l'élément de pincement (30).

3. Dispositif (1) selon l'une quelconque des revendications 1 et 2, dans lequel au moins un ressort (410, 420) est intercalé entre l'élément de base (10) et l'élément de pincement (30), et dans lequel le ressort (410, 420) fonctionne pour appliquer une force de compression constante sur les fibres (S) prises en sandwich entre l'unité de détection (20) et l'élément de pincement (30).

4. Dispositif (1) selon la revendication 3, dans lequel le ressort (410, 420) est interchangeable pour régler la force de compression appliquée aux fibres (S).

5. Dispositif (1) selon l'une quelconque des revendications 1 à 4, dans lequel une largeur ($B_1$) d'une région dans l'élément de base (10) où est montée l'unité de détection (20) est plus grande qu'une largeur ($B_2$) de l'unité de détection (20).

6. Dispositif (1) selon l'une quelconque des revendications 1 à 5, le dispositif (1) étant conçu avec une capacité de connexion filaire ou sans fil à un équipement externe (50) en vue du stockage et/ou du traitement des données délivrées par l'unité de détection (20).

7. Dispositif (1) selon l'une quelconque des revendications 1 à 6, dans lequel au moins une partie de l'unité de détection (20) et/ou de l'élément de pincement (30) venant en contact avec les fibres (S) est constituée d'un matériau qui n'est pas sensible à la génération de forces électrostatiques quand le dispositif (1) est déplacé par rapport aux fibres (S).

## FIG. 1

*FIG. 2*

*FIG. 3*

*FIG. 4*

## FIG. 5

## FIG. 6

## FIG. 7

| hair type | operator-1 | | operator-2 | |
|---|---|---|---|---|
| | average | SD | average | SD |
| natural | 0.328 | 0.022 | 0.318 | 0.017 |
| slightly bleached | 0.363 | 0.014 | 0.360 | 0.026 |
| medium bleached | 0.393 | 0.020 | 0.391 | 0.019 |

*FIG. 8*

| hair type | day | average | SD | p-value |
|---|---|---|---|---|
| natural | Day-1 | 0.330 | 0.024 | 0.846 |
| | Day-2 | 0.328 | 0.022 | |
| slightly bleached | Day-1 | 0.368 | 0.020 | 0.469 |
| | Day-2 | 0.363 | 0.014 | |
| medium bleached | Day-1 | 0.394 | 0.035 | 0.951 |
| | Day-2 | 0.393 | 0.020 | |

*FIG. 9*

| model | average | SD |
|---|---|---|
| model-1 | 0.103 | 0.009 |
| model-2 | 0.117 | 0.006 |
| model-3 | 0.154 | 0.013 |

*FIG. 10*

| application | average | SD |
|---|---|---|
| 1sh + conditioner | 0.240 | 0.008 |
| 1sh | 0.297 | 0.016 |
| 3sh | 0.299 | 0.023 |
| 5sh | 0.304 | 0.021 |
| 10sh | 0.318 | 0.017 |

## FIG. 11

| fiber | average | SD |
|---|---|---|
| paper | 0.297 | 0.016 |
| cotton cloth | 0.345 | 0.041 |
| vinyl sheet | 0.613 | 0.057 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004158526 A
- JP 2004159830 A
- JP 2009201812 A
- JP 2012239615 B